# EUROPEAN PATENT SPECIFICATION

(11) **EP 4 229 054 B1**
(45) Date of publication and mention of the grant of the patent: **04.09.2024**
(21) Application number: 21791303.7
(22) Date of filing: 11.10.2021
(51) Int. Cl.: C07D 471/04, C07D 487/04

(54) **PROCESS FOR THE PREPARATION OF 1-METHYL-6-[6-R2-5-METHYL-8-(METHYLAMINO)-4-[ (3AS,6AS)-5-METHYL-2,3,3A,4,6,6A-HEXAHYDROPYRROLO[2,3-C]PYRROL-1-YL]-9H-PYRIDO[2,3-B]INDOL-3-YL]-4-OXO-1,8-NAPHTHYRIDINE-3-CARBOXYLIC ACID HYDROCHLORIDE**
VERFAHREN ZUR HERSTELLUNG VON 1-METHYL-6-[(6-R2-5-METHYL-8-(METHYLAMINO)-4- (3AS,6AS)-5-METHYL-2,3,3A,4,6,6A-HEXAHYDROPYRROLO[2,3-C!PYRROL-1-YL)-9H-PYRIDO[2,3-B!INDOL-3-YL!-4-OXO-NAPHTHYR-3-CARBONSÄUREHYDROCHLORID
PROCÉDÉ DE PRÉPARATION DE CHLORHYDRATE D'ACIDE 1-MÉTHYL-6-[6-R2-5-MÉTHYL-8-(MÉTHYLAMINO)-4-[(3 AS, 6 AS)-5-MÉTHYL -2,3,3 A,4,6,6 A-HEXAHYDROPYRROLO[2,3-C]PYRROL-1-YL]-9 H-PYRIDO[2,3-B]INDOL-3-YL]-4-OXO-1,8-NAPHTYRIDINE-3-CARBOXYLIQUE

(30) Priority: 13.10.2020 WO PCT/CN2020/120639
(43) Date of publication of application: 23.08.2023
(73) Proprietor: F. Hoffmann-La Roche AG, 4070 Basel (CH)
(72) Inventor: CHEN, Weichun, Shanghai, 201203 (CN); LIANG, Xing, Shanghai, 201203 (CN); ZHANG, Guocai, Shanghai, 201203 (CN)
(74) Representative: Salud, Carlos E.
(86) International application number: PCT/EP2021/077975
(87) International publication number: WO 2022/078923

(56) References cited:
- WO-A1-2018/178041
- WO-A1-2020/064661
- WO-A1-2020/104436
- WO-A1-2020/109190

## Description

### Process for the preparation of 1-methyl-6-[6-R2-5-methyl-8-(methylamino)-4-[ (3aS,6aS)-5-methyl-2,3,3a,4,6,6a-hexahydropyrrolo[2,3-c]pyrrol-1-yl]-9H-pyrido[2,3-b]indol-3-yl]-4-oxo-1,8-naphthyridine-3-carboxylic acid hydrochloride

The present invention relates to a process for the preparation of compounds of formula (Ia), 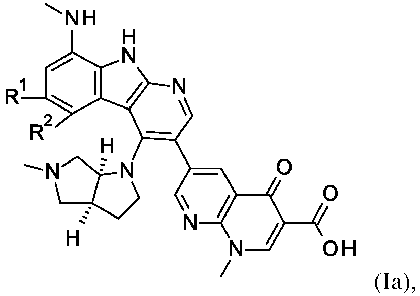 particularly compounds of formula (I) 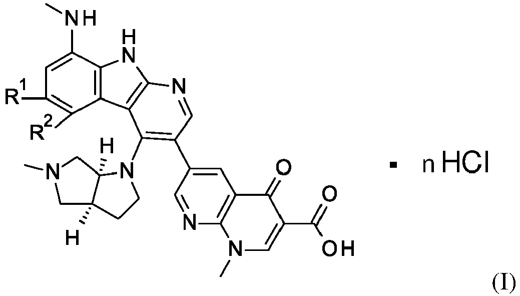 wherein R¹ is hydrogen or halogen; R² is hydrogen, halogen or cyano; n is 0-7, particularly n is 2-3, more particularly n is 2; or diastereomer, pharmaceutically acceptable salts thereof, which is useful for prophylaxis and treatment of a disease caused by bacteria infection.

### BACKGROUND OF THE INVENTION

The patent WO2018178041 disclosed synthetic approaches to obtain compounds of formula (Ia). However, the synthetic approaches disclosed in patent WO2018178041 are not suitable for large scale production due to the following issues:
(a) there are four steps to prepare final pharmaceutically active compound of formula (Ia), among which two steps involve the deprotection of Boc group and hydrolysis of ester group with low yield (58%).
(b) column purification is needed for two of the intermediates synthesis, such as: tert-butyl (3-chloro-5-cyano-6-fluoro-4-((3aS,6aS)-5-methylhexahydropyrrolo [3,4-b]pyrrol-1(2H)-yl)-9H-pyrido[2,3-b]indol-8-yl)(methyl)carbamate and ethyl 6-(8-((tert-butoxycarbonyl)(methyl)amino)-5-cyano-6-fluoro-4-((3aS,6aS) -5-methylhexahydropyrrolo[3,4-b]pyrrol-1(2H)-yl)-9H-pyrido[2,3-b]indol-3-yl)-1-methyl-4-oxo-1,4-dihydro-1,8-naphthyridine-3-carboxylate.
(c) no efficient isolation and purification method for final compound is available, current process must reply on preparative HPLC.
(d) the stability issue of free based compound of formula (Ia) makes it unsuitable for being used directly in further formulation.

One object of the invention therefore is to find an efficient synthetic approach which can be applied on a technical scale.

### FIELD OF THE INVENTION

Based on current invention of new process, above issues were nicely addressed and further advantages were provided as following;
1. HCl salt of compound of formula (I) was identified as stable form for ideal product delivery to address the stability issue from the free base API for large scale production.
2. Synthetic route was shortened from 4 steps to 3 steps with simplified synthesis and improved yield by developing new building block synthesis.
3. Column purification and HPLC purification were avoided to achieve high quality API delivery in large scale.

Another aspect of the present invention relates to a novel intermediate of compound (V):

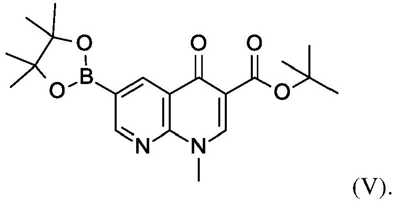

Compare to compound (Va) which is used in prior patent WO2018178041 , compound (V) used in this invention is the key intermediate in the synthesis and manufacture of pharmaceutically active compound of formula (Ia) and/or (I) as described herein. The tert-butyl ester group in compound of formula (V) can perfectly avoid the basic hydrolysis step and can be removed together with Boc group in one step. Herein, the four-step synthesis was reduced to three steps.

Another aspect of the present invention relates the salt formation of compound (Ia). After systematic screen of different salt formation, HCl salt of compound (Ia) was selected because of the excellent stability and efficient formation and isolation property.

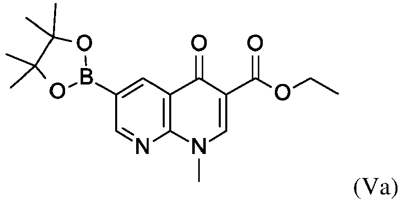

### DETAILED DESCRIPTION OF THE INVENTION

### DEFINITIONS

As used herein, the term "halogen" signifies fluorine, chlorine, bromine or iodine, particularly fluorine or chlorine.

The term "diastereomer" denotes a stereoisomer with two or more centers of chirality and whose molecules are not mirror images of one another.

The term "pharmaceutically acceptable salt" refers to conventional acid-addition salts or base-addition salts that retain the biological effectiveness and properties of the compounds of formula I and are formed from suitable non-toxic organic or inorganic acids or organic or inorganic bases. Acid-addition salts include for example those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, and the like. Base-addition salts include those derived from ammonium, potassium, sodium and, quaternary ammonium hydroxides, such as for example, tetramethyl ammonium hydroxide. The chemical modification of a pharmaceutical compound into a salt is a technique well known to pharmaceutical chemists in order to obtain improved physical and chemical stability, hygroscopicity, flow ability and solubility of compounds. It is for example described in Bastin R.J., et al., Organic Process Research & Development 2000, 4, 427-435; or in Ansel, H., et al., In: Pharmaceutical Dosage Forms and Drug Delivery Systems, 6th ed. (1995), pp. 196 and 1456-1457.

### ABBREVIATION

- ACN: Acetonitrile
- API: Active pharmaceutical ingredient
- Boc: tert-butyloxycarbonyl
- eq: Equivalent
- CataC-Pd G2: Chloro[(di(1-adamantyl)-N-butylphosphine)-2-(2-aminobiphenyl)]palladium(II), cataCXium^{®} A Pd G2
- DBU: 1,8-Diazabicyclo[5.4.0]undec-7-ene
- DCM: Dichloromethane
- DIPEA: N,N-Diisopropylethylamine
- DMAc: Dimethylacetamide
- DMF: Dimethylformamide
- DMSO: Dimethyl sulfoxide
- EA: Ethyl acetate
- HPLC: High Performance Liquid Chromatography
- IPA: Isopropanol
- IPAc: Isopropyl acetate
- MeTHF: 2-Methyltetrahydrofuran
- MSA: Methanesulfonic acid
- MTBE: Methyl tert-butyl ether
- TEA: Triethylamine
- TFA: Trifluoroacetic acid
- THF: Tetrahydrofuran
- UPLC: Ultra Performance Liquid Chromatography
- V: volume
- wt%: weight percentage

The present invention provides a process for preparing the compounds of formula (I) as outlined in the scheme 1 exemplified for the compound with R¹ is fluorine; R² is fluorine or nitrile group.

### Scheme 1

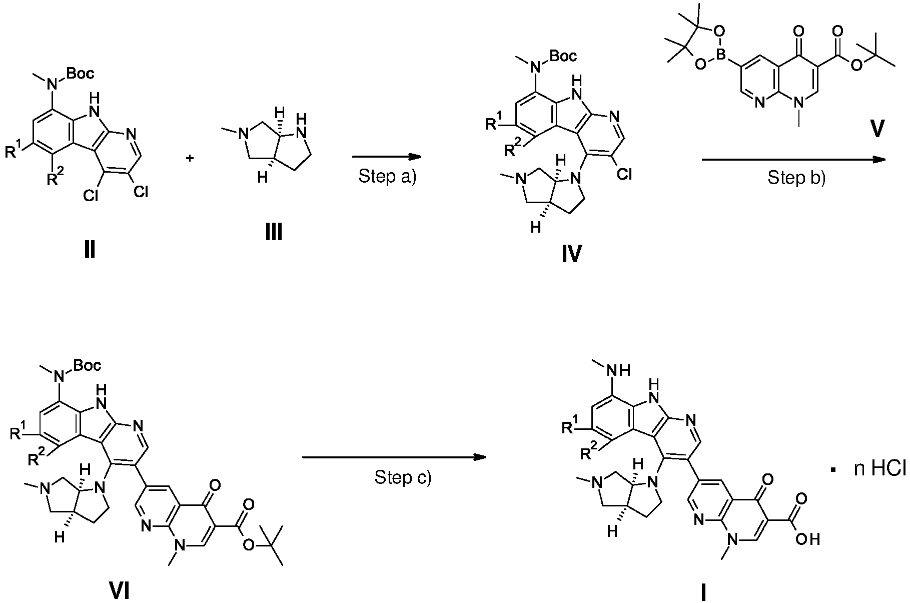

The whole synthesis comprises the following steps:
Step a) the formation of compound of formula (IV), 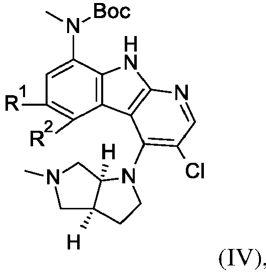 via nucleophilic aromatic substitution between compound of formula (II), 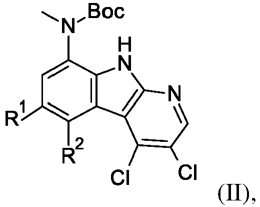 and compound (III), 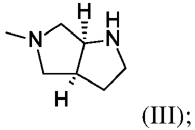
Step b) the formation of compound of formula (VI), 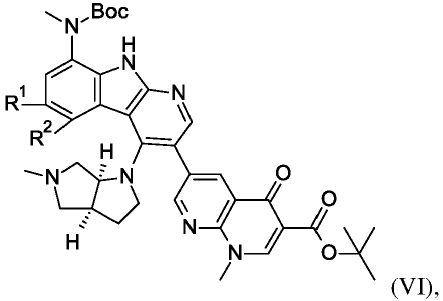 via Suzuki-Miyaura coupling reaction between compound of formula (IV) and compound (V), 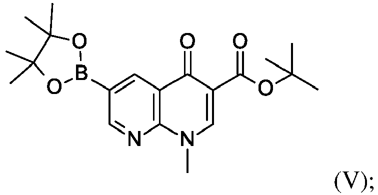
Step c) the formation of compound of formula (I), 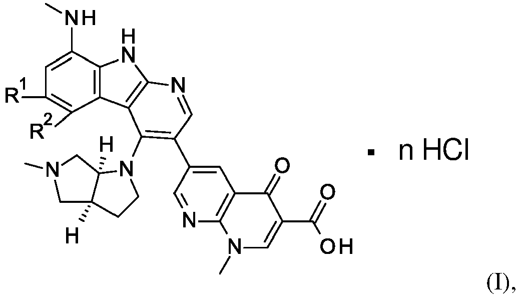 via deprotection of formula (VI); wherein R¹, R² and n are defined as above.

Another embodiment of this invention is that compound of formula (Ia) can also be synthesized in analogy to Scheme 1 after neutralization of the compound of formula (I).

A detailed description of present invention of process steps is as following:
Step a) the formation of compound of formula (IV), 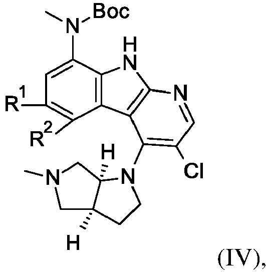 via nucleophilic aromatic substitution between compound of formula (II), 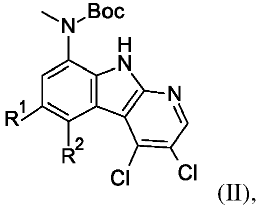 and compound (III),

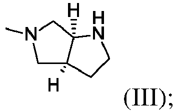

The formation of compound of formula (IV) is usually performed in the presence of a suitable base in a suitable organic solvent, followed by a recrystallization procedure. The conversion as a rule is performed under a heating condition.

The suitable base is selected from TEA, DIPEA, DBU, pyridine, K₂CO₃ and Cs₂CO₃; particularly the base is TEA.

The suitable organic solvent is selected from DMF, DMSO, DMAc, Toluene, DCM, CHCl₃, benzene, THF, MeTHF, IPA, t-BuOH and ACN; particularly the organic solvent is ACN.

The reaction is performed at 20 °C - 120 °C; particularly at 75 °C - 80 °C.

The solid formation of the product via crystallization can be performed in the same solvent system as used in the reaction. The suitable solvent used both in the reaction and the crystallization is a mixture of ACN and the base, wherein the base is selected from TEA and DIPEA, particularly the base is TEA. The suitable volume ratio of TEA/ACN is 1/20 to pure TEA; particularly the volume ratio is 1/5.

In prior art (e.g., WO2018178041), DMSO is used as the solvent. DMSO containing reaction mixture has potential explosion risk and could decompose at high temperature which makes it not a good solvent for scale-up. In addition, column purification is required during the work up stage , however recrystallization in the solvent of ACN/TEA in this step of present invention could surprisingly address above issue, which also results in cleaner reaction and a work-up procedure without solvent change.

Step b) the formation of compound of formula (VI), 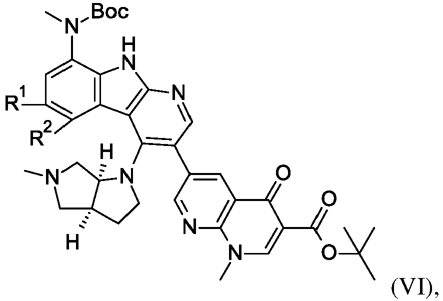 via Suzuki-Miyaura coupling reaction between compound of formula (IV) and compound (V),

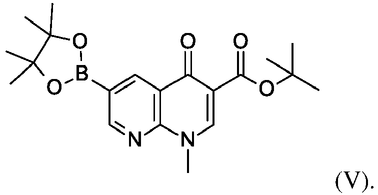

This step is critical for the whole process in terms of yield and purity improvement. In prior art (e.g., WO2018178041), a compound (Va) was used with additional two more steps to deprotect Boc group and hydrolyze the ester group with tedious isolation and column purification procedure. In present invention, compound (V) was used and ester hydrolysis step was successfully avoided.

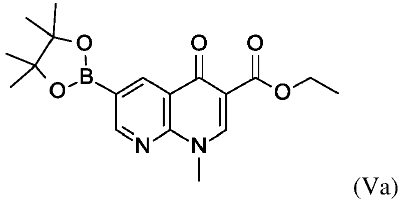

In prior art (e.g., WO2018178041), flash column chromatography was used, which cannot meet the requirement of large scale production. In present invention, different purification and isolation methods were tried, however direct crystallization of the crude compound of formula (VI) under various conditions was never successful which either gave no precipitation or precipitation with low purity or low yield. Finally, the acid-base workup procedure with carefully selected pH value and solvent system surprisingly gives an efficient and reliable process for technical scale production. Following recrystallization surprisingly gives more than 99% percent purity product with high yield (more than 80%).

The formation of compound of formula (VI) is synthesized in the presence of a suitable catalyst and a suitable base in a suitable solvent. The conversion as a rule is performed under a heating condition.

The suitable catalyst is selected from Palladium(II) analogues with Phosphine Ligands, Nickel Catalyst and Palladium Precatalyst; particularly the catalyst is XPhos Pd G2, SPhos Pd G2, P(Cy3)Pd G3, APhos Pd G3, cataC-Pd G2 and cataC-Pd G3; more particularly the catalyst is cataC-Pd G2.

The suitable base used in the reaction is selected from NaOtBu, KOtBu, NaOH, KOH, MeONa, MeOK, Cs₂CO₃, K₂CO₃, K₃PO₄, KHCO₃, Na₂CO₃ and NaHCO₃; particularly the base is K₂CO₃ or Na₂CO₃.

The suitable solvent used in the reaction is a mixture of water and an organic solvent. The organic solvent is selected from MeTHF, THF, Dioxane, Toluene, Benzene, DMF, DMSO, DMAc, DCM, CHCl₃, IPA, MeOH and EtOH; particularly the solvent is Dioxane.

The ratio of water to the organic solvent is surprisingly important to this reaction. Reducing the amount of water and increasing reaction temperature will unpredictably increase the yield and the purity of product. The suitable volume ratio of water to organic solvent is from 1/2 to 1/100; particularly the ratio is 1/40.

The reaction is performed at 20°C - 110°C; particularly at 90°C - 100°C.

The purification of compound of formula (VI) is achieved via an acid-base work-up with a suitable acid and base in a suitable solvent at a suitable final PH; and recrystallization of formula (VI) is performed in a suitable organic solvent.

The acid used in the acid-base work-up is selected from HCl, HBr, H₂SO₄, H₃PO₄, MSA, toluene sulfonic acid and camphor sulfonic acid, particularly the acid is HCl. The base used in the acid-base work-up is selected from NaOH, KOH, KHCO₃, K₂CO₃, NaHCOs and Na₂CO₃; particularly the base is NaOH. The suitable final pH range is from 0 to 10; particularly the pH is 4 to 5.

The suitable solvent for acid-base work-up is a selected from EtOH, MeOH, THF, IPAc, MTBE, EA, Toluene, benzene and DCM; particular the organic solvent is DCM and Toluene.

The suitable solvent for recrystallization of compound of formula (VI) was selected from acetone, ACN, MeOH, EtOH and IPA; particularly the solvent is EtOH.

Step c) the formation of compound of formula (I), 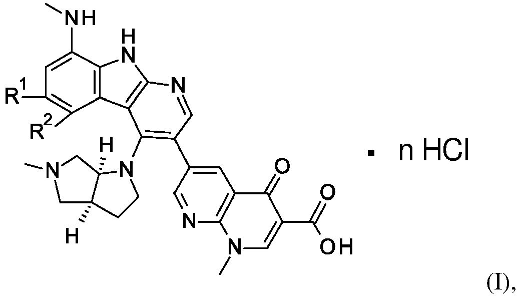 via deprotection of formula (VI).

In present invention, the deprotection of Boc group and hydrolysis of ester group is performed in one step. The compound of formula (I) is synthesized in the presence of a suitable acid in a suitable organic solvent.

The suitable acid used in the reaction is selected from TFA, HCl, H₃PO₄ and CH₃COOH and HCOOH; particularly the acid is HCl.

The organic solvent is selected from MeTHF, THF, DCM, CHCl₃, EA, IPAc, IPA, MeOH and EtOH; particularly the solvent is THF or EA.

In another embodiment, the present invention relates to the synthetic process of 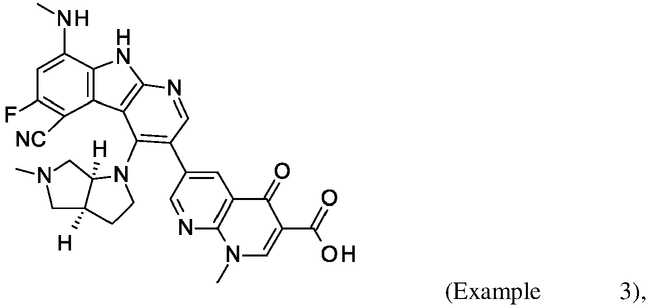 and its salt

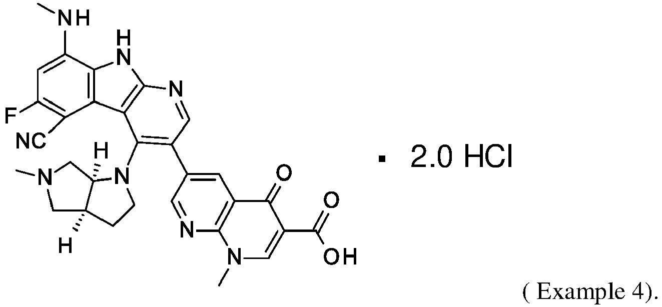

In another embodiment, the present invention relates to the synthetic process of 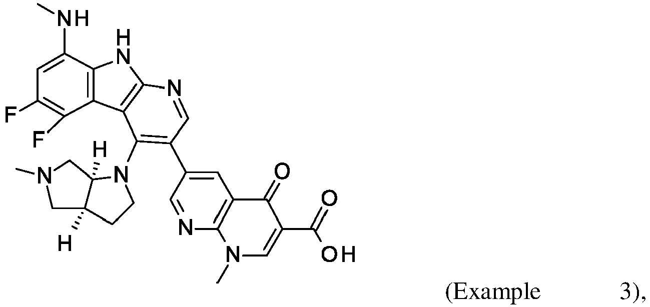 and its salt

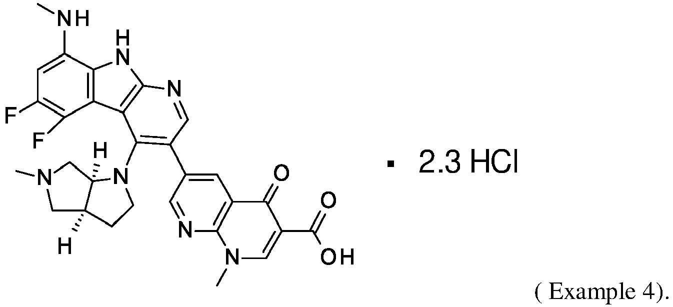

### SUMMARY OF THE INVENTION

The present invention relates to (i) process for the preparation of a compound of the formula (I), 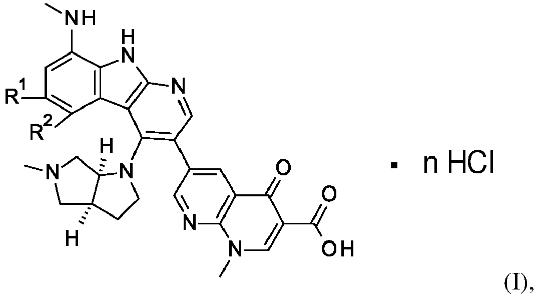
wherein R¹ is hydrogen or halogen; R² is hydrogen, halogen or cyano; n is 0-7, particularly n is 2-3, more particularly n is 2;
comprising any of the following steps:
   Step a) the formation of compound of formula (IV), 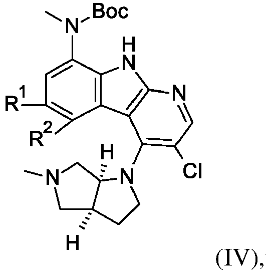 via nucleophilic aromatic substitution between compound of formula (II), 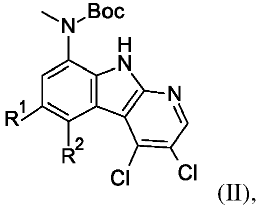 and compound (III), 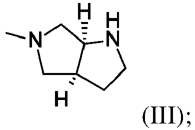
   Step b) the formation of compound of formula (VI), 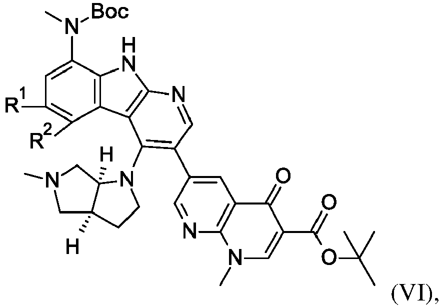 via Suzuki-Miyaura coupling reaction between compound of formula (IV) and compound (V), 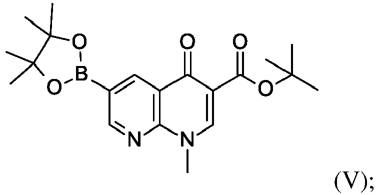
   Step c) the formation of compound of formula (I), 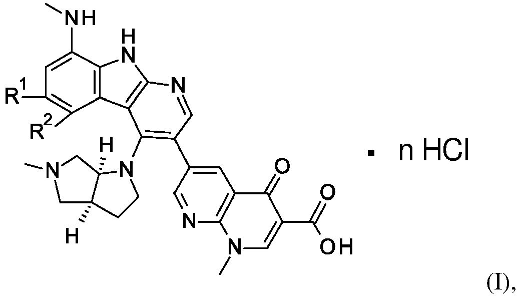 via deprotection of formula (VI).

A further embodiment of present invention is (ii) the process according to (i), wherein R¹ is hydrogen, fluoro or chloro; R² is hydrogen, fluoro, chloro or cyano.

A further embodiment of present invention is (iii) the process according to (i) to (ii), wherein the formation of compound of formula (IV) in step a) is performed in the presence of a base in an organic solvent; wherein the base is selected from TEA, DIPEA, DBU, pyridine, K₂CO₃ and Cs₂CO₃ particularly the base is TEA; wherein the solvent which is selected from DMF, DMSO, DMAc, Toluene, DCM, CHCl₃, benzene, THF, MeTHF, IPA, t-BuOH and ACN, particularly the organic solvent is ACN.

A further embodiment of present invention is (iv) the process according to (i) to (iii), wherein the suitable volume ratio of TEA/ACN is 1/20 to pure TEA; particularly the volume ratio is 1/5.

A further embodiment of present invention is (v) the process according to (i) to (iv), wherein the formation of compound of formula (VI) in step b) is performed in the presence of a catalyst, a base in a solvent; wherein the catalyst is selected from Palladium(II) analogues with Phosphine Ligands, Nickel Catalyst and Palladium Precatalyst; particularly the catalyst is XPhos Pd G2, SPhos Pd G2, P(Cy3)Pd G3, APhos Pd G3, cataC-Pd G2 and cataC-Pd G3; more particularly the catalyst is cataC-Pd G2.

A further embodiment of present invention is (vi) the process according to (i) to (v), wherein the base is selected from NaOtBu, KOtBu, NaOH, KOH, MeONa, MeOK, Cs₂CO₃, K₂CO₃, K₃PO₄, KHCO₃, Na₂CO₃ and NaHCO₃; particularly the base is K₂CO₃ or Na₂CO₃.

A further embodiment of present invention is (vii) the process according to (i) to (vii), wherein the solvent is a mixture of water and an organic solvent, wherein the organic solvent is selected from MeTHF, THF, Dioxane, Toluene, Benzene, DMF, DMSO, DMAc, DCM, CHCl₃, IPA, MeOH and EtOH; particularly the solvent is Dioxane.

A further embodiment of present invention is (viii) the process according to (i) to (vii), wherein ratio of water to the organic solvent is 1/2 to 1/100; particularly the ratio is 1/40.

A further embodiment of present invention is (ix) the process according to (i) to (viii), wherein the compound of formula (VI) was purified via an acid-base work-up process in a solvent at a final PH; wherein the acid used in the process is selected from HCl, HBr, H₂SO₄, H₃PO₄, MSA, toluene sulfonic acid and camphor sulfonic acid, particularly the acid is HCl; wherein the base used in the process is selected from NaOH, KOH, KHCO₃, K₂CO₃, NaHCOs and Na₂CO₃; particularly the base is NaOH.

A further embodiment of present invention is (x) the process according to (i) to (ix), wherein the solvent is selected from EtOH, MeOH, THF, IPAc, MTBE, EA, Toluene, benzene and DCM; particular the organic solvent is DCM and Toluene.

A further embodiment of present invention is (xi) the process according to (i) to (x), wherein the final PH range is from 0 to 10; particularly the pH is 4 to 5.

A further embodiment of present invention is (xii) the process according to (i) to (xi), wherein the compound of formula (VI) was further recrystallized in a solvent after acid-base work-up , wherein the solvent is selected from acetone, ACN, MeOH, EtOH and IPA; particularly the solvent is EtOH.

A further embodiment of present invention is (xiii) the process according to (i) to (xii), the formation of compound of formula (I) in step c) is performed in the presence of an acid in a solvent; wherein the acid is selected from TFA, HCl, H₃PO₄ and CH₃COOH and HCOOH, particularly the acid is HCl; wherein solvent is selected from MeTHF, THF, DCM, CHCl₃, EA, IPAc, IPA, MeOH and EtOH, particularly the solvent is THF or EA.

### EXAMPLES

### Example 1

### tert-Butyl N-[3-chloro-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate

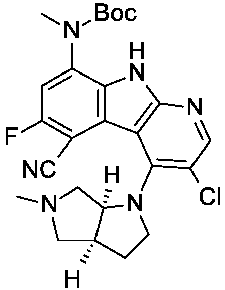

To a 500m L glass flask was charged (3,4-dichloro-5-cyano-6-fluoro-9H-pyrido[2,3-b]indol-8-yl)(methyl)carbamate (20 g, 46.4 mmol), (3aS, 6aS)-5-methyloctahydropyrrolo[3,4-b]pyrrole (9.25 g, 69.6 mmol, compound (III)), TEA (29 g, 40 mL) and ACN (44 g, 100 mL). The reaction mixture was heated to 80 °C - 85 °C and stirred for 20 hours.

After reaction completion, the reaction mixture was cooled to 20 °C - 25 °C over 5 hours. The resulting suspension was stirred at 20 °C - 25 °C for 2 hours, then filtered and the solid was collected and washed with ACN (10mL, three times). Combine and concentrate the organic solution and the solid was recrystallization in 50mL ACN and 20mL TEA. After filtration, the solid was collected. Two portion of wet solid was combine and dried in vacuum oven to afford 19.4 g of tert-butyl N-[3-chloro-5-cyano-6-fluoro-4-[ (3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate (Example 1). The purity was 98.2%, the yield was 82.1 %, and MS m/e = 499.3[M+H] +.

### Example 2

### 1-Methyl-4-oxo-6-(4,4,5,5-tetramethyl-1,3,2-dioxaborolan-2-yl)-1,4-dihydro-1,8-naphthyridine-3-carboxylate

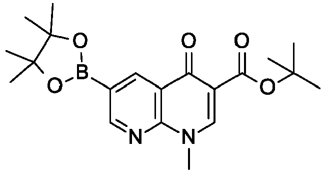

The Compound (V) was prepared according to following scheme:

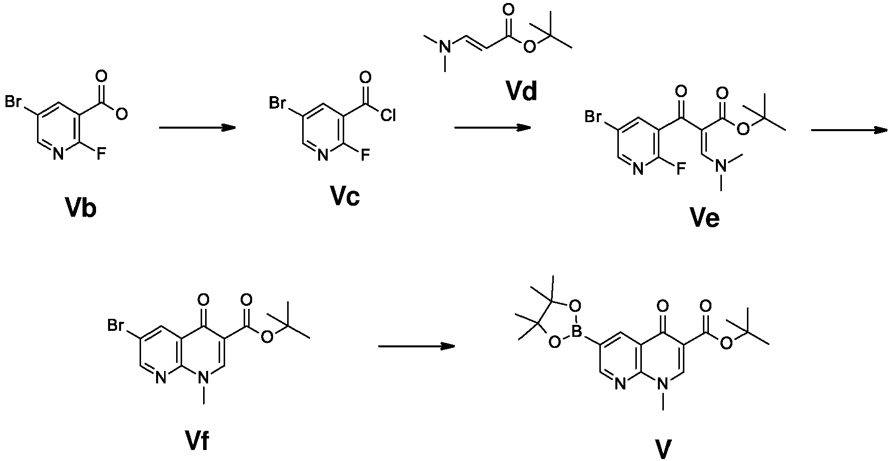

### Step 1) preparation of 5-bromo-2-fluoronicotinoyl chloride (Compound (Vc))

To a 1000 m L three neck glass flask with N₂ protected was charged 5-bromo-2-fluoronicotinic acid (25 g, 110 mmol, 1 eq), DCM (330 g, 250 mL), DMF (66 g, 50 mL) and oxalyl chloride (23.6 g, 16.2 mL, 182 mmol, 1.65 eq). The mixture was stirred at 20 °C over 16 hours. The result mixture was concentrated under vacuum to afford crude product of formula (Vc) (26.3 g, 110 mmol, 100 % yield). The crude product was used in the next step directly.

### Step 2) preparation of tert-butyl(Z)-2-(5-bromo-2-fluoronicotinoyl)-3-(dimethylamino)acrylate (Compound Ve)

To a 1000 m L three neck glass flask with N₂ protected was charged tert-butyl (E)-3-(dimethylamino)acrylate (19.9 g, 110 mmol, 1 eq), toluene (318 g, 368 mL) and Et3N (22.3 g, 30.7 mL, 221 mmol, 2 eq). The mixture was heated to 80 °C and 5-bromo-2-fluoronicotinoyl chloride (26.3 g, 110 mmol, 1 eq) was added. The result mixture was stirred at 80 °C 3 hours.

After reaction completion, the reaction mixture was cooled to room temperature. The resulting mixture was poured to 300 mL of ice water. After phase separation, the organic layer was washed by 300 mL of brine twice and then dried over Na₂SO₄. The organic layer was concentrated and mixed with n-heptane (54 g, 78.9 mL). The result suspension was filtered and the filter cake was dried under vacuum overnight. Tert-butyl (Z)-2-(5-bromo-2-fluoronicotinoyl)-3-(dimethylamino)acrylate (35.0 g, 93.8 mmol, 85 % yield) was obtained as a pale-yellow powder.

### Step 3) preparation of tert-butyl 6-bromo-1-methyl-4-oxo-1,4-dihydro-1,8-naphthyridine-3-carboxylate (Compound Vf)

To a 1000 m L three neck glass flask with N₂ protection was charged tert-butyl (Z)-2-(5-bromo-2-fluoronicotinoyl)-3-(dimethylamino)acrylate (35 g, 93.8 mmol, 1 eq), THF (216 g, 245 mL) and DBU (15.7 g, 15.5 mL, 103 mmol). The mixture was cooled to 0 °C and methanamine (51.6 mL, 103 mmol, 1.1 eq) was added. The result mixture was stirred at 0 °C for 2 hours.

After reaction was completed, the reaction mixture was concentrated under vacuum. The resulting residue was mixed with ethanol (138 g, 175 mL) and water (35 g, 35 mL). The resulting suspension was filtered and the filter cake was washed by 35 mL water, dried under vacuum, and tert-butyl 6-bromo-1-methyl-4-oxo-1,4-dihydro-1,8-naphthyridine-3-carboxylate (25.2 g, 74.3 mmol, 79.2 % yield) was obtained.

### Step 4) preparation of tert-butyl 1-methyl-4-oxo-6-(4,4,5,5-tetramethyl-1,3,2-dioxaborolan-2-yl)-1,4-dihydro-1,8-naphthyridine-3-carboxylate (Compound (V))

To a 1000 in L three neck glass flask with N₂ protection was charged with Pd₂(dba)₃ (675 mg, 737 µmol, 0.01 eq), xPhos (1.76 g, 3.69 mmol, 0.05 eq), and toluene (303 g, 350 mL). The result mixture was stirred at room temperature for 1 hour. KOAc (21.7 g, 221 mmol, 3 eq), tert-butyl 6-bromo-1-methyl-4-oxo-1,4-dihydro-1,8-naphthyridine-3-carboxylate (25 g, 73.7 mmol, e1 eq), 4,4,4',4',5,5,5',5'-octamethyl-2,2'-bi(1,3,2-dioxaborolane) (25.1 g, 95.8 mmol, 1.3 eq) and toluene (303 g, 350 mL) were added into the mixture. The result mixture was stirred at 90 °C for 3 hours.

After reaction was completed, the reaction mixture was cooled to room temperature and concentrated under vacuum. The resulting suspension was filtered and the filter cake was washed by 50mL DCM and 50mL toluene mixture. The filtrate was concentrated to give the crude product. And the crude product was triturated in 300 mL of MTBE/ Heptane (v/v=1/1) at room temperature for 2 hours, then filtered. And the filter cake was washed with 50 mL of MTBE/heptane(v/v=2/1) and 25 mL heptane, then dried under vacuum to give tert-butyl 1-methyl-4-oxo-6-(4,4,5,5-tetramethyl-1,3,2-dioxaborolan-2-yl)-1,4-dihydro-1,8-naphthyridine-3-carboxylate (22 g, 57 mmol, 77.3 % yield) as an off-white solid.
MS (ESI): 387.3([M+H]⁺). **¹H NMR** (300 MHz, CDCl₃) δ ppm 9.16 (d, *J*=1.5 Hz, 1 H), 9.01 (d, *J*=1.8 Hz, 1 H), 8.53 (s, 1 H), 3.97 (s, 3 H), 1.62 (s, 9 H), 1.37 (s, 12 H)

### Example 3

### tert-Butyl 6-[8-[tert-butoxycarbonyl(methyl)amino]-5-cyano-6-fluoro-4-[ (3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate

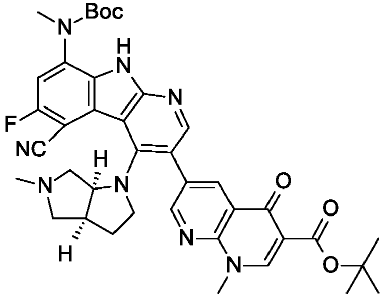

To a 1000 m L three neck glass flask with N₂ protection was charged with tert-butyl (3-chloro-5-cyano-6-fluoro-4-((3aS,6aS)-5-methylhexahydropyrrolo[3,4-b]pyrrol-1(2H)-yl)-9H-pyrido[2,3-b]indol-8-yl)(methyl)carbamate (18g, 36.1 mmol) and tert-butyl 1-methyl-4-oxo-6-(4,4,5,5-tetramethyl-1,3,2-dioxaborolan-2-yl)-1,4-dihydro-1,8-naphthyridine-3-carboxylate (22.3 g, 57.7 mmol), CataC-Pd G2 (1.21 g, 1.8 mmol), 1,4-dioxane (360 mL), potassium carbonate (15 g, 108 mmol) and water (15 mL). The reaction mixture was heated to 95 °C - 100 °C and stirred for 1 hour.

After reaction was completed, the reaction mixture was cooled to room temperature. The resulting suspension was filtered and the filter cake was washed with 100 mL dioxane. The filtrate was concentrated and then dissolved in 200mL dioxane. The solution was added to 800 mL water slowly over 1 hour and precipitate formed. The suspension was filtered, the filter cake was washed by 100 mL water two times and dried, and then dissolved in the mixture of 150 mL DCM and 50 mL MeOH. The organic layer was extracted by 150 mL 0.1N HCl aqueous solution four times. The aqueous solution was neutralized to pH = 8 by NaOH 2N solution. Then, the aqueous solution was extracted by 200 mL, DCM three times. The combined organic layer was washed with brine, dried over Na₂SO₄, concentrated to afford crude product. The crude product was dissolved in 60ml EtOH at 60 °C and then slowly cool to room temperature over 3 hours. The precipitate was formed and was collected through filtration. The wet solid was dried in oven over 18h and afford 21.4 g of ethyl 6-[8-[tert-butoxycarbonyl(methyl)amino]-5-cyano-6-fluoro-4-[ (3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate. The purity was 98.3% and the yield was 82% and the MS (ESI): 723.3([M+H]⁺).

### Example 4

### 6-[5-Cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid; dihydrochloride

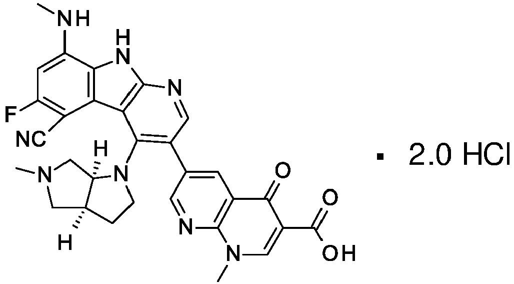

To a 1000 m L three neck glass flask with N₂ protection was charged with tert-butyl 6-(8-((tert-butoxycarbonyl)(methyl)amino)-5-cyano-6-fluoro-4-((3aS,6aS)-5-methylhexahydropyrrolo[3,4-b]pyrrol-1(2H)-yl)-9H-pyrido[2,3-b]indol-3-yl)-1-methyl-4-oxo-1,4-dihydro-1,8-naphthyridine-3-carboxylate (25 g, 34.6 mmol), THF (440 g, 500 mL) and HCl (41.5 g, 37% wt% aqueous solution, 34.5 mL, 415 mmol). The reaction mixture was stirred at room temperature for 22 hours. The reaction mixture was concentrated and then re-dissolved in 370 mL ethanol. The suspension was stirred at room temperature over 3 hours. After filtration, the wet cake was suspended in 250 mL ethanol. The result suspension was filtered and dried in oven at 50 °C over 24 hours to afford 21.0g HCl salt of 6-[5-cyano-6-fluoro-8-(methylamino)-4-[ (3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid. The purity was 98.2% and the yield was 95%. MS (ESI): 567.4 ([M+H]⁺). ¹H NMR (400 MHz, DMSO-d6) δ ppm 14.82 (br s, 1 H), 11.89 (br s, 1 H), 9.25 (s, 1 H), 9.16 (d, *J*=2.3 Hz, 1 H), 8.76 (d, *J*=2.3 Hz, 1 H), 8.03 (s, 1 H), 6.83 (br d, *J*=3.3 Hz, 1 H), 6.65 (d, *J*=13.1 Hz, 1 H), 4.60 ~ 4.74 (m, 1 H), 4.17 (s, 3 H), 2.89 ~ 3.05 (m, 5 H), 2.71 ~ 2.81 (m, 1 H), 2.46 (br d, *J*=9.0 Hz, 1 H), 2.07 ~ 2.31 (m, 6 H), 1.66 (dd, J=9.7, 5.1 Hz, 1 H), 1.42 (br s, 1 H). ¹⁹F NMR (376 MHz, DMSO-*d*₆) δ ppm -112.76 (s, 1 F). Cl ion test (Dionex Interion HPLC): 1.99 and 2.05 in two separate tests. Cl ratio; average: 2.02.

### Example 5

### tert-Butyl N-[3-chloro-5,6-difluoro-4-[ (3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate

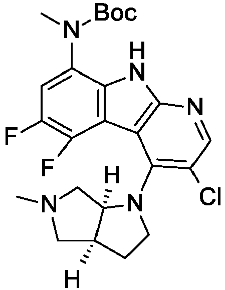

To a 1000 mL glass flask was charged with tert-butyl (3,4-dichloro-5,6-difluoro-9H-pyrido[2,3-b]indol-8-yl)(methyl)carbamate (100 g, 249 mmol), (3aS, 6aS)-5-methyloctahydropyrrolo[3,4-b]pyrrole (66.1 g, 497 mmol), Et₃N (200 mL) and ACN (400 mL). The reaction mixture was heated to 80 °C - 85 °C and stirred for 20 hours.

After reaction was completed, the reaction mixture was cooled to room temperature over 2 hours. The resulting suspension was stirred at room temperature for 2 hours, then filtered and the collected solid was washed with ACN (50 mL, three times). The wet solid was dried in vacuum oven to afford 106g of tert-butyl N-[3-chloro-5-cyano-6-fluoro-4-[ (3aS,6aS)-5-methyl-2,3,3a,4,6,6a-hexahydropyrrolo[2,3-c]pyrrol-1-yl]-9H-pyrido[2,3-b]indol-8-yl]-N-methylcarbamate (Example 1). The purity was 98.0%, the yield was 84.9 %, and MS (ESI): 492.2 ([{³⁵C1}M+H] ⁺), 494.2 ([{³⁷Cl}M+H] ⁺).

### Example 6

### Screening of Suzuki-Miyaura coupling reaction condition for the synthesis of compound of formula (VI)

The formation of Compound of formula (VI) is essential to the scale up and quality control of formula (I), which requires a comprehensive design for the choice of reaction condition to achieve optimized product recovery and quality.

In the following tests, to each 10 mL tube was added tert-butyl (3-chloro-5,6-difluoro-4-((3aS,6aS)-5-methylhexahydropyrrolo[3,4-b]pyrrol-1(2H)-yl)-9H-pyrido[2,3-b]indol-8-yl)(methyl)carbamate (100 mg, 0.189 mmol), tert-butyl 1-methyl-4-oxo-6-(4,4,5,5-tetramethyl-1,3,2-dioxaborolan-2-yl)-1,4-dihydro-1,8-naphthyridine-3-carboxylate (154 mg, 378 mmol), Cata C Xium @RA-PdG2, potassium bicarbonate (78.4 mg, 0.576 mmol), 1,4-dioxane (2 mL, 20 V) and water. The reaction mixture was heated to 100 °C and stirred for 6 hours. The result was summarized in Table 1.

**Table 1. Compound (VI) Screening result**

| Entry | water | PdG2 | Conversion | Purity (%)* |
|---|---|---|---|---|
| 1 | 0.5 v | 0.1 eq | 100% | 73 |
| 2 | 0.5 v | 0.05 eq | 97% | 83 |
| 3 | 0.2 v | 0.05 eq | 97% | 88 |
| 4 | 2 v | 0.1 eq | 62% | - |
| 5 | 2 v | 0.05 eq | 55% | - |

| | | | | |
|---|---|---|---|---|
| * the product purity was tested by UPLC | | | | |

Based on the above data, lower Pd catalyst (entry 2 compare to entry 1) has cleaner product. Lower water content (entry 3 compare to entry 2 and entry 5) has better conversion and purity. So, 0.2v water and 0.05eq Pd catalyst is the best reaction condition based on current result.

### Example 7

### tert-Butyl 6-[8-[tert-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate

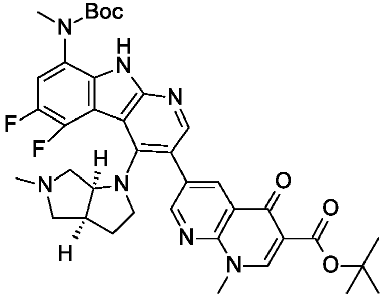

To a 1000m L three neck glass flask with N₂ protection was charged with tert-butyl (3-chloro-5,6-difluoro-4-((3aS,6aS)-5-methylhexahydropyrrolo[3,4-b]pyrrol-1(2H)-yl)-9H-pyrido[2,3-b]indol-8-yl)(methyl)carbamate (48 g, 97.6 mmol), tert-butyl 1-methyl-4-oxo-6-(4,4,5,5-tetramethyl-1,3,2-dioxaborolan-2-yl)-1,4-dihydro-1,8-naphthyridine-3-carboxylate (75.4 g, 195 mmol), CataC-Pd G2 (3.26 g, 4.88 mmol), potassium bicarbonate (29.3 g, 293 mmol), 1,4-dioxane (500 mL) and water (5 mL). The reaction mixture was heated to 95 °C - 100 °C and stirred for 18 hours.

After reaction was completed, the reaction mixture was cooled to room temperature. The resulting suspension was concentrated and then dissolved in 1 L ethanol and 2 L 0.1N HCl aqueous solution. The solution was washed by 2 L toluene three times and then added to 800 mL water slowly over 1 hour and precipitate was formed. The suspension was filtered and washed by 100 mL water two times. The filter cake was dried and then dissolved in 150 mL DCM and 50 mL MeOH. The organic layer was extracted by 150mL 0.1N HCl aqueous solution four times. The aqueous solution was neutralized to pH = 8 by NaOH 2N solution. Then, the aqueous solution was extracted by 200 mL DCM three times. The combined DCM layer was washed with brine and dried over Na₂SO₄, concentrated to afford crude product. The crude product was dissolved in 80mL EtOH at 60 °C and then slowly cool to room temperature over 2 hours. The precipitate was formed and was collected through filtration. The wet solid was dried in oven over 18h and afford 57.2 g of tert-butyl 6-[8-[tert-butoxycarbonyl(methyl)amino]-5,6-difluoro-4-[ (3aR,6aR)-5-methyl-3,3a,4,5,6,6a-hexahydro-2H-cyclopenta[b]pyrrol-1-yl]-9H-pyrido[2,3-b]indol-3-yl]-1-methyl-4-oxo-1,8-naphthyridine-3-carboxylate. The purity is 98.3% and the yield is 82% and the MS (ESI): 715.4 ([M+H]⁺).

### Example 8

### 6-[5,6-Difluoro-8-(methylamino)-4-[ (3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid; dihydrochloride

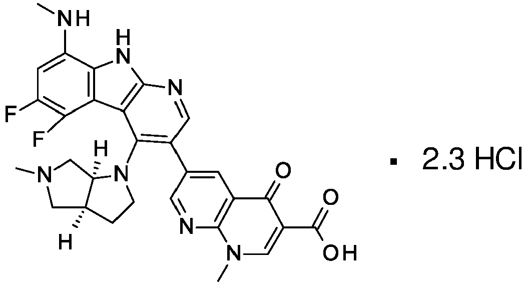

To a 1000 m L three neck glass flask with N₂ protection was charged with tert-butyl 6-[8-[tert-butoxycarbonyl(methyl)amino]-5,6-difluoro-4-[ (3aR,6aR)-5-methyl-3,3a,4,5,6,6a-hexahydro-2H-cyclopenta[b]pyrrol-1-yl]-9H-pyrido[2,3-b]indol-3-yl]-1-methyl-4-oxo-1,8-naphthyridine-3-carboxylate (47 g, 65.7 mmol), THF (500 mL) and HCl (131 g, 109 mL, 1.31 mol). After being stirred at room temperature for 18 hours, the reaction mixture was concentrated and then dissolved in 500 mL ethanol and 100 mL water. The suspension was heated to 60 °C over 2 hours and then slowly cooled to room temperature over 3 hours. After filtration, the filter cake was dissolved in 400 mL ethanol and suspension formed. The result suspension was filtered and dried in oven at 50 °C over 24 hours to afford 43.5 g HCl salt of 6-[5-cyano-6-fluoro-8-(methylamino)-4-[ (3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid. The purity is 97.8% and the yield is 94%.

¹H NMR (400 MHz, DMSO-d6) δ ppm: 14.856 (br s, 1 H), 11.716 (br s, 1 H), 9.052 ~ 9.409 (m, 2 H), 8.714 (br s, 1 H), 8.120 (s, 1 H), 6.564 ~ 6.597 (m, 1 H), 5.672 (br s, 1 H), 4.454 (br s, 1 H), 4.169 ( s, 3 H), 2.904 (m, 5 H), 2.824 (br s, 1 H), 2.510 (m, 1 H), 2.098 ~ 2.165 (m, 4 H), 1.963 (m, 1 H), 1.814 (br s, 1 H), 1.614 (br s, 1 H); MS (ESI): 560.3 ([M+H] ⁺), 280.7 ([M/2+H] ⁺). Cl ion test (Dionex Interion HPLC): 2.34 and 2.32 in two separate tests; average: 2.33.

### Example 9

### Stability experiment of final compound of formula (I)

Freebase of Example 4: 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid; initial purity: 99.16%

HCl salt compound (Example 4): 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid; hydrochloride; initial purity: 98.50%

Freebase of Example 8: 6-[5,6-difluoro-8-(methylamino)-4-[ (3aS,6aS)-5-methyl-2,3,3 a,4,6,6a-hexahydropyrrolo [2,3-c]pyrrol-1-yl] -9H-pyrido [2,3-b] indol-3-yl]-1-methyl-4-oxo-1,8-naphthyridine-3-carboxylic acid; initial purity: 97.96%

HCl salt compound (Example 8): 6-[5,6-difluoro-8-(methylamino)-4-[ (3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid; hydrochloride; initial purity: 97.80%

Condition: 10 mg of compound in 4 mL vial with sealed cap. Keep in oven at certain temperature for certain days. Then, the purity of solid was checked by UPLC. The result was summarized in Table 2:

**Table 2. Stability test of Compound (I) or (Ia)**

| Test Sample | Initial Purity | Stability Result | |
|---|---|---|---|
| | | Solid at 100 °C / 1day | Solid at 20 °C / 7day |
| Freebase of Example 4 | 99.16% | 93.35% | 95.73% |
| HCl salt compound (Example 4) | 98.50% | 98.48% | 98.67% |
| Freebase of Example 8 | 97.96% | 91.08% | 96.72% |
| HCl salt compound (Example 8) | 97.80% | 97.77% | 97.78% |

| | | | |
|---|---|---|---|
| Apparently, HCl salt compound (Example 4 and 8) are much more stable than its freebase form, which is critical to the large scale production. | | | |

## Claims

1. Process for the preparation of a compound of the formula (I), 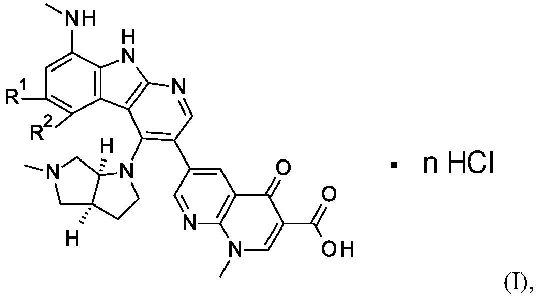
wherein R¹ is hydrogen or halogen; R² is hydrogen, halogen or cyano; n is 0-7, particularly n is 2-3, more particularly n is 2;
comprising any of the following steps:
Step a) the formation of compound of formula (IV), 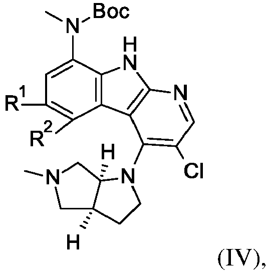 via nucleophilic aromatic substitution between compound of formula (II), 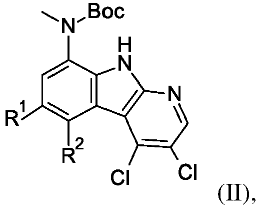 and compound (III), 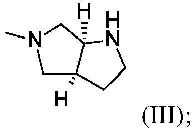
Step b) the formation of compound of formula (VI), 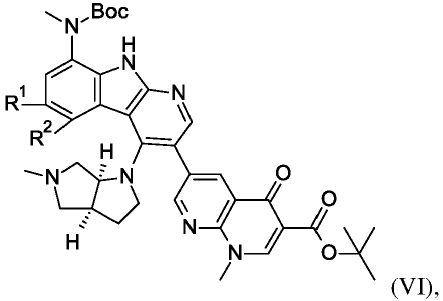 via Suzuki-Miyaura coupling reaction between compound of formula (IV) and compound (V), 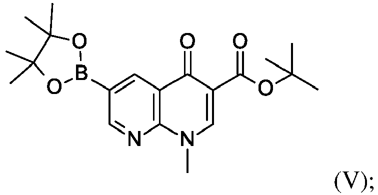
Step c) the formation of compound of formula (I), 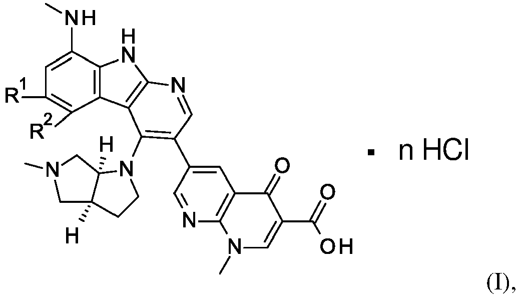 via deprotection of formula (VI).

2. A process according to claim 1, wherein R¹ is hydrogen, fluoro or chloro; R² is hydrogen, fluoro, chloro or cyano.

3. A process according to claim 1 or 2, wherein the formation of compound of formula (IV) in step a) is performed in the presence of a base in an organic solvent; wherein the base is selected from TEA, DIPEA, DBU, pyridine, K₂CO₃ and Cs₂CO₃, particularly the base is TEA; wherein the solvent is selected from DMF, DMSO, DMAc, Toluene, DCM, CHCl₃, benzene, THF, MeTHF, IPA, t-BuOH and ACN, particularly the organic solvent is ACN.

4. A process according claim 3, wherein the suitable volume ratio of TEA/ACN is 1/20 to pure TEA; particularly the volume ratio is 1/5.

5. A process according to any one of claims 1 to 4, wherein the formation of compound of formula (VI) in step b) is performed in the presence of a catalyst and a base in a solvent; wherein the catalyst is selected from Palladium(II) analogues with Phosphine Ligands, Nickel Catalyst and Palladium Precatalyst; particularly the catalyst is XPhos Pd G2, SPhos Pd G2, P(Cy3)Pd G3, APhos Pd G3, cataC-Pd G2 and cataC-Pd G3; more particularly the catalyst is cataC-Pd G2.

6. A process according claim 5, wherein the base is selected from NaOtBu, KOtBu, NaOH, KOH, MeONa, MeOK, Cs₂CO₃, K₂CO₃, K₃PO₄, KHCO₃, Na₂CO₃ and NaHCO₃; particularly the base is K₂CO₃ or Na₂CO₃.

7. A process according claim 5 or 6, wherein the solvent is a mixture of water and an organic solvent, wherein the organic solvent is selected from MeTHF, THF, Dioxane, Toluene, Benzene, DMF, DMSO, DMAc, DCM, CHCl₃, IPA, MeOH and EtOH; particularly the solvent is Dioxane.

8. A process according claim 7, wherein ratio of water to the organic solvent is 1/2 to 1/100; particularly the ratio is 1/40.

9. A process according any one of claims 5-8, wherein the compound of formula (VI) was purified via an acid-base work-up process in a solvent at a final PH; wherein the acid used in the process is selected from HCl, HBr, H₂SO₄, H₃PO₄, MSA, toluene sulfonic acid and camphor sulfonic acid, particularly the acid is HCl; wherein the base used in the process is selected from NaOH, KOH, KHCO₃, K₂CO₃, NaHCO₃ and Na₂CO₃; particularly the base is NaOH.

10. A process according to claim 9, wherein the solvent is selected from EtOH, MeOH, THF, IPAc, MTBE, EA, Toluene, benzene and DCM; particular the organic solvent is DCM and Toluene.

11. A process according to claims 9 or 10, wherein the final PH range is from 0 to 10; particularly the pH is 4 to 5.

12. A process according any one of claims 5-11, wherein the compound of formula (VI) was further recrystallized in a solvent after acid-base work-up , wherein the solvent is selected from acetone, ACN, MeOH, EtOH and IPA; particularly the solvent is EtOH.

13. A process according any one of claims 1-12, the formation of compound of formula (I) in step c) is performed in the presence of an acid in a solvent; wherein the acid is selected from TFA, HCl, H₃PO₄ and CH₃COOH and HCOOH, particularly the acid is HCl; wherein solvent is selected from MeTHF, THF, DCM, CHCl₃, EA, IPAc, IPA, MeOH and EtOH, particularly the solvent is THF or EA.

## Patentansprüche

1. Verfahren zur Herstellung einer Verbindung der Formel (I), 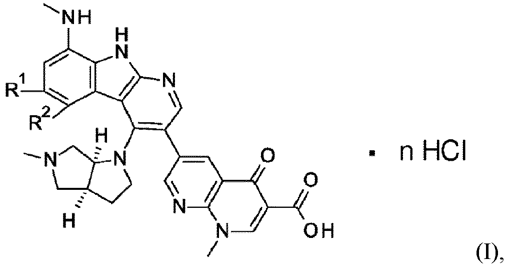
wobei R¹ Wasserstoff oder Halogen ist; R² Wasserstoff, Halogen oder Cyano ist; n 0-7 ist, n insbesondere 2-3 ist, n speziell 2 ist;
umfassend einen der folgenden Schritte:
Schritt a) die Bildung der Verbindung der Formel (IV), 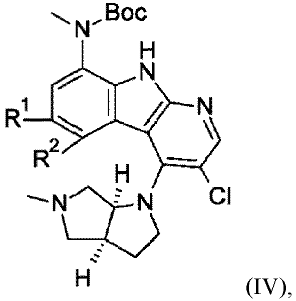 durch nucleophile aromatische Substitution zwischen der Verbindung der Formel (II), 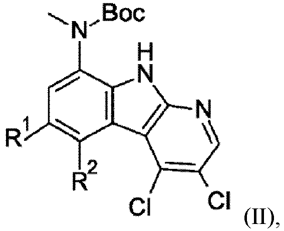 und der Verbindung (III), 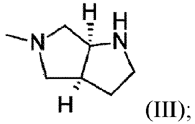
Schritt b) die Bildung der Verbindung der Formel (VI), 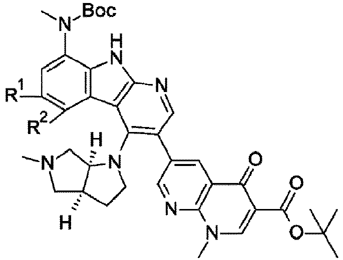 (VI), durch Suzuki-Miyaura-Kupplungsreaktion zwischen der Verbindung der Formel (IV) und der Verbindung (V), 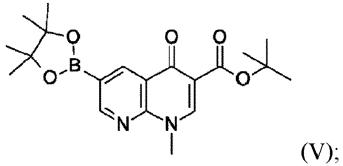
Schritt c) die Bildung der Verbindung der Formel (I), 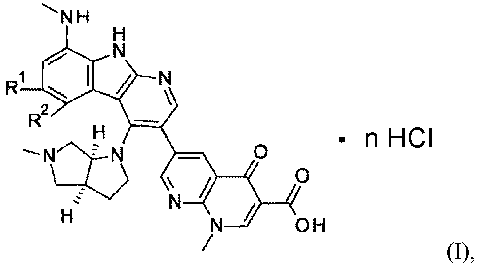 durch Entschützung der Formel (VI).

2. Verfahren nach Anspruch 1, wobei R¹ Wasserstoff, Fluor oder Chlor ist; R² Wasserstoff, Fluor, Chlor oder Cyano ist.

3. Verfahren nach Anspruch 1 oder 2, wobei die Bildung der Verbindung der Formel (IV) in Schritt a) in Gegenwart einer Base in einem organischen Lösungsmittel durchgeführt wird; wobei die Base ausgewählt ist aus TEA, DIPEA, DBU, Pyridin, K₂CO₃ und Cs₂CO₃, die Base insbesondere TEA ist; wobei das Lösungsmittel ausgewählt ist aus DMF, DMSO, DMAc, Toluol, DCM, CHCl₃, Benzol, THF, MeTHF, IPA, t-BuOH und ACN, das organische Lösungsmittel insbesondere ACN ist.

4. Verfahren nach Anspruch 3, wobei das geeignete Volumenverhältnis von TEA/ACN von 1:20 zu bis zu reinem TEA ist; das Volumenverhältnis insbesondere 1:5 ist.

5. Verfahren nach einem der Ansprüche 1 bis 4, wobei die Bildung der Verbindung der Formel (VI) in Schritt b) in Gegenwart eines Katalysators und einer Base in einem Lösungsmittel durchgeführt wird; wobei der Katalysator ausgewählt ist aus Palladium(II)-Analoga mit Phosphinliganden, Nickelkatalysator und Palladium-Präkatalysator; der Katalysator insbesondere XPhos Pd G2, SPhos Pd G2, P(Cy3)Pd G3, APhos Pd G3, cataC-Pd G2 und cataC-Pd G3 ist; der Katalysator speziell cataC-Pd G2 ist.

6. Verfahren nach Anspruch 5, wobei die Base ausgewählt ist aus NaOtBu, KOtBu, NaOH, KOH, MeONa, MeOK, Cs₂CO₃, K₂CO₃, K₃PO₄, KHCO₃, Na₂CO₃ und NaHCO₃; die Base insbesondere K₂CO₃ oder Na₂CO₃ ist.

7. Verfahren nach Anspruch 5 oder 6, wobei das Lösungsmittel eine Mischung aus Wasser und einem organischen Lösungsmittel ist, wobei das organische Lösungsmittel ausgewählt ist aus MeTHF, THF, Dioxan, Toluol, Benzol, DMF, DMSO, DMAc, DCM, CHCl₃, IPA, MeOH und EtOH; das Lösungsmittel insbesondere Dioxan ist.

8. Verfahren nach Anspruch 7, wobei das Verhältnis von Wasser zu dem organischen Lösungsmittel 1:2 bis 1:100 ist; das Verhältnis insbesondere 1:40 ist.

9. Verfahren nach einem der Ansprüche 5 bis 8, wobei die Verbindung der Formel (VI) über ein Säure-Base-Aufarbeitungsverfahren in einem Lösungsmittel bei einem End-PH-Wert gereinigt wurde; wobei die in dem Verfahren verwendete Säure ausgewählt ist aus HCl, HBr, H₂SO₄, H₃PO₄, MSA, Toluolsulfonsäure und Kampfersulfonsäure, die Säure insbesondere HCl ist; wobei die in dem Verfahren verwendete Base ausgewählt ist aus NaOH, KOH, KHCO₃, K₂CO₃, NaHCO₃ und Na₂CO₃; die Base insbesondere NaOH ist.

10. Verfahren nach Anspruch 9, wobei das Lösungsmittel ausgewählt ist aus EtOH, MeOH, THF, IPAc, MTBE, EA, Toluol, Benzol und DCM; das organische Lösungsmittel insbesondere DCM und Toluol ist.

11. Verfahren nach Anspruch 9 oder 10, wobei der End-pH-Bereich von 0 bis 10 reicht; der pH-Wert insbesondere 4 bis 5 ist.

12. Verfahren nach einem der Ansprüche 5 bis 11, wobei die Verbindung der Formel (VI) nach der Säure-Base-Aufarbeitung weiter in einem Lösungsmittel umkristallisiert wurde, wobei das Lösungsmittel ausgewählt ist aus Aceton, ACN, MeOH, EtOH und IPA; das Lösungsmittel insbesondere EtOH ist.

13. Verfahren nach einem der Ansprüche 1 bis 12, die Bildung der Verbindung der Formel (I) in Schritt c) in Gegenwart einer Säure in einem Lösungsmittel durchgeführt wird; wobei die Säure ausgewählt ist aus TFA, HCl, H₃PO₄ und CH₃COOH und HCOOH, die Säure insbesondere HCl ist; wobei das Lösungsmittel ausgewählt ist aus MeTHF, THF, DCM, CHCl₃, EA, IPAc, IPA, MeOH und EtOH, das Lösungsmittel insbesondere THF oder EA ist.

## Revendications

1. Procédé de préparation d'un composé de formule (I), 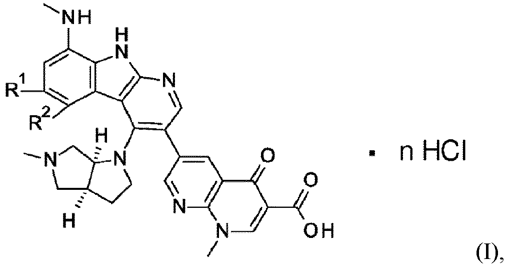
dans lequel R¹ représente un hydrogène ou un halogène ; R² représente un hydrogène, un halogène ou un cyano ; n représente de 0 à 7, en particulier n représente de 2 à 3, plus particulièrement n représente 2 ;
comprenant l'une quelconque des étapes suivantes :
étape a) la formation du composé de formule (IV), 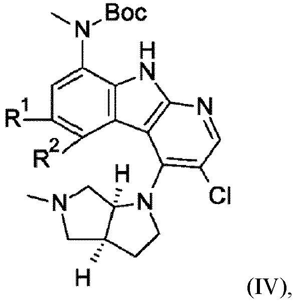 par l'intermédiaire d'une substitution aromatique nucléophile entre le composé de formule (II), 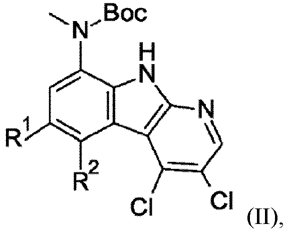 et le composé (III), 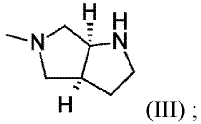 étape b) la formation du composé de formule (VI), 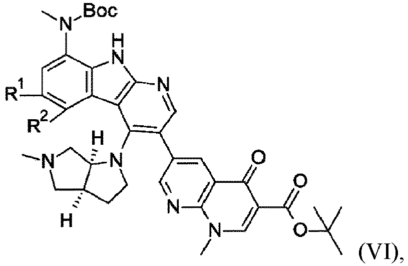 par l'intermédiaire d'une réaction de couplage de Suzuki-Miyaura entre le composé de formule (IV) et le composé (V), 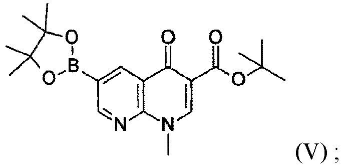
étape c) la formation du composé de formule (I), 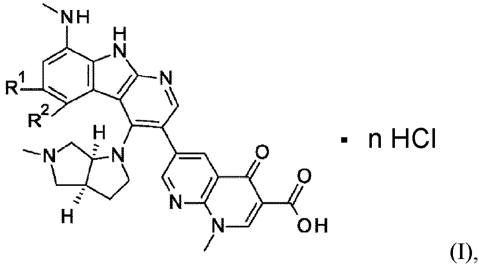 par l'intermédiaire d'une déprotection de la formule (VI).

2. Procédé selon la revendication 1, dans lequel R¹ représente un hydrogène, un fluor ou un chlore ; R² représente un hydrogène, un fluor, un chlore ou un cyano.

3. Procédé selon la revendication 1 ou 2, dans lequel la formation du composé de formule (IV) à l'étape a) est réalisée en présence d'une base dans un solvant organique ; dans lequel la base est choisie parmi la TEA, la DIPEA, le DBU, la pyridine, le K₂CO₃ et le Cs₂CO₃, en particulier la base est la TEA ; dans lequel le solvant est choisi parmi le DMF, le DMSO, le DMAc, le toluène, le DCM, le CHCl₃, le benzène, le THF, le MeTHF, l'IPA, le t-BuOH et l'ACN, en particulier le solvant organique est l'ACN.

4. Procédé selon la revendication 3, dans lequel le rapport de volume de TEA/ACN approprié est de 1/20 à la TEA pure ; en particulier le rapport de volume est de 1/5.

5. Procédé selon l'une quelconque des revendications 1 à 4, dans lequel la formation du composé de formule (VI) à l'étape b) est réalisée en présence d'un catalyseur et d'une base dans un solvant ; dans lequel le catalyseur est choisi parmi des analogues du palladium(II) avec des ligands phosphine, un catalyseur au nickel et un précatalyseur au palladium ; en particulier le catalyseur est le XPhos Pd G2, le SPhos Pd G2, le P(Cy3)Pd G3, l'APhos Pd G3, le cataC-Pd G2 et le cataC-Pd G3 ; plus particulièrement le catalyseur est le cataC-Pd G2.

6. Procédé selon la revendication 5, dans lequel la base est choisie parmi le NaOtBu, le KOtBu, le NaOH, le KOH, le MeONa, le MeOK, le Cs₂CO₃, le K₂CO₃, le K₃PO₄, le KHCO₃, le Na₂CO₃ et le NaHCOs ; en particulier la base est le K₂CO₃ ou le Na₂CO₃.

7. Procédé selon la revendication 5 ou 6, dans lequel le solvant est un mélange d'eau et d'un solvant organique, dans lequel le solvant organique est choisi parmi le MeTHF, le THF, le dioxane, le toluène, le benzène, le DMF, le DMSO, le DMAc, le DCM, le CHCl₃, l'IPA, le MeOH et l'EtOH ; en particulier le solvant est le dioxane.

8. Procédé selon la revendication 7, dans lequel le rapport de l'eau au solvant organique est de 1/2 à 1/100 ; en particulier le rapport est de 1/40.

9. Procédé selon l'une quelconque des revendications 5 à 8, dans lequel le composé de formule (VI) a été purifié par l'intermédiaire d'un procédé de traitement acide-base dans un solvant à un pH final ; dans lequel l'acide utilisé dans le procédé est choisi parmi l'HCl, l'HBr, l'H₂SO₄, l'H₃PO₄, le MSA, l'acide toluènesulfonique et l'acide camphresulfonique, en particulier l'acide est HCl ; dans lequel la base utilisée dans le procédé est choisie parmi le NaOH, le KOH, le KHCO₃, le K₂CO₃, le NaHCO₃ et le Na₂CO₃ ; en particulier la base est le NaOH.

10. Procédé selon la revendication 9, dans lequel le solvant est choisi parmi l'EtOH, le MeOH, le THF, l'IPAc, le MTBE, l'EA, le toluène, le benzène et le DCM ; en particulier le solvant organique est le DCM et le toluène.

11. Procédé selon les revendications 9 ou 10, dans lequel la plage de pH final est de 0 à 10 ; en particulier le pH est de 4 à 5.

12. Procédé selon l'une quelconque des revendications 5 à 11, dans lequel le composé de formule (VI) a été en outre recristallisé dans un solvant après le traitement acide-base, dans lequel le solvant est choisi parmi l'acétone, l'ACN, le MeOH, l'EtOH et l'IPA ; en particulier le solvant est l'EtOH.

13. Procédé selon l'une quelconque des revendications 1 à 12, la formation du composé de formule (I) à l'étape c) est réalisée en présence d'un acide dans un solvant ; dans lequel l'acide est choisi parmi le TFA, l'HCl, l'H₃PO₄ et le CH₃COOH et l'HCOOH, en particulier l'acide est l'HCl ; dans lequel le solvant est choisi parmi le MeTHF, le THF, le DCM, le CHCl₃, l'EA, l'IPAc, l'IPA, le MeOH et l'EtOH, en particulier le solvant est le THF ou l'EA.
